(19) Europäisches Patentamt
European Patent Office
Office européen des brevets

(11) Numéro de publication: **0 243 336 B1**

(12) # FASCICULE DE BREVET EUROPEEN

(45) Date de publication du fascicule du brevet: **13.06.90**

(51) Int. Cl.⁵: **A61K 31/72**
// (A61K31/72, 31:40)

(21) Numéro de dépôt: **87870049.1**

(22) Date de dépôt: **10.04.87**

(54) **Compositions pharmaceutiques pour le traitement de la claudication intermittente.**

(30) Priorité: **14.04.86 GB 8609033**

(43) Date de publication de la demande: **28.10.87 Bulletin 87/44**

(45) Mention de la délivrance du brevet: **13.06.90 Bulletin 90/24**

(84) Etats contractants désignés: **AT BE CH DE ES FR GB GR IT LI LU NL SE**

(56) Documents cités:
**DE-A-3 030 863**
**FR-A-2 236 513**

(73) Titulaire: **U C B, S.A., 326, Avenue Louise, B-1050 Bruxelles(BE)**

(72) Inventeur: **Gobert, Jean, 120, rue du Cornet, B-1040 Bruxelles(BE)**
Inventeur: **Verloes, René, 159A, Meersstraat, B-1742 Ternat(BE)**

(74) Mandataire: **Dusseldorp, Raymond et al, U.C.B. S.A. Département D.T.B. 326, avenue Louise, Bte 7, B-1050 Bruxelles(BE)**

**EP 0 243 336 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à des compositions pharmaceutiques et plus particulièrement à des compositions pharmaceutiques contenant des hydroxyéthyl-amidons et des composés à activité nootrope, à la préparation de ces compositions, de même qu'à leur utilisation dans le traitement de la claudication intermittente.

Au cours de ces dernières années, on s'est servi de plus en plus de substituts de plasma, et parmi ceux-ci en particulier d'hydroxyéthyl-amidons, pour le traitement par hémodilution isovolémique et hypervolémique des troubles de la circulation périphérique non opérables.

Ainsi, par exemple, dans la demande de brevet allemand 3.030.863, on décrit l'utilisation d'hydroxyéthyl-amidons sous la forme d'une perfusion pour le traitement des troubles de la circulation. Ces hydroxyéthyl-amidons ont un poids moléculaire compris entre 10.000 et 250.000, de préférence entre 30.000 et 80.000 et sont employés sous la forme d'une solution aqueuse contenant 5 à 13, de préférence 8 à 12% d'hydroxyéthyl-amidon. Une augmentation nette de la pression d'oxygène tissulaire est observée par la perfusion de 500 ml d'une solution à 10% d'un hydroxyéthyl-amidon d'un poids moléculaire de 40.000. D'après cette demande de brevet, ce traitement provoque une diminution significative de la viscosité du plasma et de l'agrégation des érythrocytes, de même qu'une amélioration de la déformabilité des érythrocytes en sorte que la microcirculation du sang s'en trouve sensiblement améliorée. Par ailleurs, des essais cliniques ont montré que les solutions d'hydroxyéthyl-amidon peuvent être utilisées avec succès, entre autres pour allonger la distance de marche sans douleur dans le traitement de la claudication intermittente chez des patients atteints de troubles circulatoires.

La demanderesse vient présentement de faire la découverte tout à fait surprenante et imprévisible que l'effet thérapeutique des hydroxyéthyl-amidons dans le traitement de la claudication intermittente est potentialisé par les composés à activité nootrope.

En fait, les composés à activité nootrope sont déjà utilisés dans le domaine médical en raison de leur activité sur le système nerveux central qui leur confère une action bénéfique sur les facultés d'acquisition et de rétention de la mémoire et sur les fonctions mentales en général. A l'heure actuelle, ces composés sont surtout utilisés pour le traitement des insuffisances cérébrales, des troubles de la mémoire et des difficultés tant de concentration mentale que d'apprentissage. Mais, à la connaissance de la demanderesse, la littérature n'enseigne pas que ces composés soient utiles dans le traitement de la claudication intermittente.

La présente invention a donc pour objet de nouvelles compositions pharmaceutiques qui se caractérisent en ce qu'elles contiennent au moins un hydroxyéthyl-amidon et au moins un composé à activité nootrope et, le cas échéant, un ou plusieurs excipients ou diluants pharmaceutiques non toxiques.

De façon plus spécifique, la présente invention a pour objet des compositions pharmaceutiques telles que définies ci-dessus et conditionnées en vue de leur usage médical dans le traitement de la claudication intermittente.

Parmi les composés à activité nootrope, on peut avantageusement utiliser dans le but visé par la présente invention, les composés répondant à la formule générale

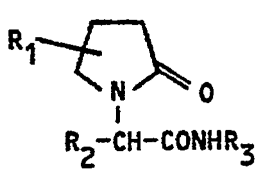

(I)

dans laquelle
$R_1$ représente un atome d'hydrogène ou un groupe hydroxyl,
$R_2$ un atome d'hydrogène ou un radical alkyle inférieur et
$R_3$ un atome d'hydrogène, le radical $-CH_2CONH_2$, le radical 2-[bis(1-méthyléthyl)amino]éthyle ou un radical de formule

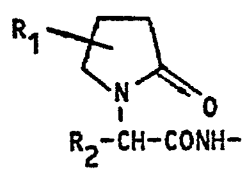

dans laquelle $R_1$ et $R_2$ ont la même signification que ci-dessus.

Les composés précités peuvent se présenter, le cas échéant, sous la forme de mélanges stéréoisomères, de racémates ou d'isomères optiquement actifs purs. Conformément à la présente invention, les composés à activité nootrope peuvent donc aussi être utilisés avantageusement sous ces différentes

formes, en vue de renforcer l'action thérapeutique des hydroxyéthyl-amidons dans le traitement de la claudication intermittente.

Les composés répondant à la formule I sont des substances connues pour leur activité sur le système nerveux central, en particulier pour leur activité nootrope. Lorsque $R_1$, $R_2$ et $R_3$ représentent des atomes d'hydrogène, le composé répondant à la formule générale I est le 2-oxo-1-pyrrolidineacétamide (ou piracétam). Ce composé et sa préparation font l'objet du brevet britannique 1.039.113.

Lorsque $R_1$ et $R_3$ représentent des atomes d'hydrogène et $R_2$ un radical alkyle inférieur, par exemple le radical éthyle, les composés répondant à la formule générale I sont des alpha-alkyl-2-oxo-1-pyrrolidineacétamides, par exemple l'alpha-éthyl-2-oxo-1-pyrrolidineacétamide (ou étiracétam). Ces composés et leur préparation font l'objet du brevet britannique 1.309.692. Ces derniers composés peuvent aussi se présenter sous la forme d'isomères optiquement actifs. Ces composés et leur préparation font l'objet des demandes de brevet européen 165.919 et 162.036. Lorsque $R_1$ et $R_2$ représentent des atomes d'hydrogène et $R_3$ le radical $-CH_2CONH_2$, le composé répondant à la formule générale I est le 2-(2-oxo-1-pyrrolidineacétamido)-acétamide. Ce composé et sa préparation font l'objet du brevet belge 59.925. Lorsque $R_1$ et $R_2$ représentent des atomes d'hydrogène et $R_3$ le radical

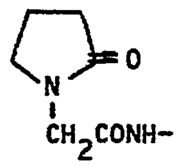

le composé répondant à la formule générale I est le 2'-[(2-oxo-pyrrolidino)-acétyl]-2-oxo-1-pyrrolidinoacétohydrazide (ou dupracétam). Ce composé et sa préparation font l'objet du brevet belge 838.880. Lorsque $R_1$ et $R_2$ représentent des atomes d'hydrogène et $R_3$ le radical 2-[bis(1-méthyléthyl) amino]éthyle, le composé répondant à la formule générale I est le N-[2-[bis (1-méthyléthyl)amino]éthyl]-2-oxo-1-pyrrolidineacétamide (ou pramiracétam). Ce composé et sa préparation font l'objet du brevet américain 4.145.347. Enfin, lorsque $R_2$ et $R_3$ représentent des atomes d'hydrogène et $R_1$ un groupe hydroxyle occupant la position 4 dans le radical 2-oxo-1-pyrrolidinyle, le composé répondant à la formule générale I est le 4-hydroxy-2-oxo-1-pyrrolidineacétamide (ou oxiracétam). Ce composé et sa préparation font l'objet du brevet américain 4.118.396.

Comme autre exemple de composé à activité nootrope, on peut citer la 1-(p-méthoxybenzoyl)-2-pyrrolidinone (ou aniracétam) qui fait l'objet du brevet européen 5143.

Les hydroxyéthyl-amidons que l'on peut utiliser conformément à la présente invention sont les hydroxyéthyl-amidons couramment employés dans la pratique médicale en tant que substituts du plasma. Ces produits sont obtenus par hydroxyéthylation d'amidons trés riches en amylopectine (plus de 90%), qui proviennent de variétés spéciales de maïs, de sorgho ou de riz, modifiées génétiquement, connues également sous la dénomination anglo-saxonne "waxy starch". Conformément à l'invention, ces hydroxyéthyl-amidons possédent utilement un poids moléculaire compris entre 30.000 (en abrégé HES 30) et 450.000 (en abrégé HES 450), de préférence un poids moléculaire d'environ 200.000 (en abrégé HES 200). La substitution molaire (MS) de ces hydroxyéthyl-amidons peut varier entre environ 0,3 et 0,9. Par substitution molaire, on entend le nombre moyen de moles de groupes hydroxyéthyle présents par mole de résidus d'anhydroglucose de l'amidon.

Selon une caractéristique avantageuse de l'invention, ces compositions pharmaceutiques se présentent sous forme de solutions ou suspensions aqueuses contenant 5 à 13% en poids, de préférence 8 à 12% en poids d'hydroxyéthyl-amidon et 0,6 à 3% en poids de composé à activité nootrope.

Le rapport en poids entre l'hydroxyéthyl-amidon et le composé à activité nootrope est de 1,6 : 1 à 21,6 : 1, de préférence de 2,7 : 1 à 20 : 1.

La préparation des compositions pharmaceutiques conformes à l'invention est effectuée selon des méthodes connues en soi, par mélange, de préférence en solution ou suspension aqueuse, d'un hydroxyéthyl-amidon et d'un composé à activité nootrope appropriés, ces composés étant présents dans le rapport en poids cité ci-dessus. Le cas échéant, on peut y ajouter un ou plusieurs excipients ou diluants pharmaceutiques non toxiques.

L'administration des compositions pharmaceutiques conformes à l'invention est effectuée de préférence par voie intraveineuse ou intraartérielle sous la forme d'une perfusion unique ou de perfusions multiples, ou encore sous la forme d'une perfusion continue.

Pour le traitement de la claudication intermittente chez l'homme, il est particulièrement recommandé d'administrer deux fois à trois fois par semaine à 2 ou 3 jours d'intervalle, sous la forme d'une perfusion, une dose de composé à activité nootrope comprise entre 3 et 15 g mise en solution ou en suspension dans une solution aqueuse contenant 5 à 13% en poids, de préférence 8 à 12% en poids, d'hydroxyéthyl-amidon. En outre, dans les intervalles de temps sans perfusion, on administrera utilement une dose supplémentaire de 3 à 12 g par jour de composé à activité nootrope, par exemple sous la forme d'une suspension ou d'une solution buvable aqueuse.

Ainsi qu'on va le montrer dans l'exemple de mise en oeuvre donné ci-après, des résultats particulièrement favorables ont été obtenus en utilisant le 2-oxo-1-pyrrolidineacétamide (ou piracétam), produit réputé pour son activité nootrope.

Chez des patients présentant des troubles de la micro- ou macrocirculation avec des oblitérations ou des microangiopathies des membres inférieurs, on a pu démontrer un effet de potentialisation marqué du piracétam sur l'action thérapeutique des hydroxyéthyl-amidons. En effet, lors d'essais cliniques comparatifs d'une durée de six semaines et dans lesquels ces patients ont été traités par hémodilution d'abord isovolémique, puis hypervolémique avec 500 ml d'une solution à 10% de HES 200, on a observé que l'addition d'une dose de 12 g de piracétam à cette solution augmente de manière trés significative la distance moyenne de marche sans douleur, par rapport à celle obtenue dans des conditions expérimentales identiques, mais sans addition de piracétam. On a constaté, en outre, une nette diminution du temps de claudication et des douleurs.

Parallèlement au traitement, on a déterminé avant et après celui-ci la fluidité du sang des patients étudiés. Avant le traitement, la fluidité du sang de tous les patients était trés limitée. après le traitement, il s'est avéré que les patients ayant reçu du piracétam en même temps que le HES 200, avaient la fluidité sanguine la meilleure.

L'intérêt médical de la présente invention est donc évident puisqu'il permet de renforcer considérablement l'action thérapeutique des hydroxyéthyl-amidons dans la thérapie de la claudication intermittente et de celle résultant d'une artériopathie oblitérante périphérique en particulier.

L'exemple donné ci-après illustre la présente invention.

Exemple.

Cet exemple décrit un essai clinique effectué sur deux groupes A et B de 9 patients présentant une artériopathie oblitérante périphérique aux stades cliniques IIa et IIb. Tous ces patients présentaient des oblitérations d'un ou plusieurs jours ou des microangiopathies des membres inférieurs. En outre, la fluidité du sang de ces patients était trés limitée.

Les patients du groupe A (âge moyen: 60 ans) reçoivent un traitement combiné par dilution isovolémique et dilution hypervolémique au moyen de HES 200 (poids moléculaire 200.000) et de piracétam.

A cet effet, en 4 à 5 séances, on prélève chaque fois 500 ml de sang que l'on remplace par une perfusion isovolémique de 500 ml d'une solution aqueuse stérile à 10% de HES 200 contenant 12 g de piracétam. Le rapport en poids entre le HES 200 et le piracétam est donc de 4,1 : 1.

Entre chaque séance de perfusion isovolémique s'écoule un intervalle de 2 à 3 jours pendant lequel les patients reçoivent 12 g de piracétam par jour sous forme d'une suspension buvable. Lors de ces séances, l'hématocrite est tombé à des valeurs comprises entre 35 et 38%.

Ensuite, on administre trois fois par semaine, en perfusion hypervolémique, 500 ml de la solution à 10% de HES 200 contenant 12 g de piracétam.

Le traitement dure six semaines au total et après le traitement, on accepte un hématocrite maximal de 46%.

Le groupe B constitue le groupe témoin et les patients de ce groupe (âge moyen: 60 ans) reçoivent le même traitement, mais sans addition de piracétam.

Le tableau suivant indique la distance moyenne (exprimée en mètres) de marche sans douleur, mesurée sur le tapis de marche, avant et après le traitement.

Tableau

Distance moyenne de marche sans douleur (en m).

| | groupe A (HES + piracétam) | groupe B (HES) |
|---|---|---|
| Avant traitement | 178 | 186 |
| Après traitement | 297 | 255 |
| Allongement (en %) | 67% | 37% |

L'examen de ce tableau montre qu'un allongement de la distance moyenne de marche sans douleur s'observe après le traitement, aussi bien parmi les patients du groupe témoin B que parmi les patients du groupe traité A. Toutefois, il apparaît clairement que l'allongement de la distance de marche sans douleur est cliniquement plus important (67%) dans le groupe traité avec l'association HES 200 - piracétam que dans le groupe témoin (37%) traité avec le HES seul; cette différence est statistiquement significative ($p < 0,05$).

Dans les deux groupes de patients, on a également suivi l'évolution de divers paramètres hémorhéologiques. On a observé ainsi qu'en ajoutant le piracétam au HES 200, la fluidité du sang est sensiblement

améliorée dans le sens d'une diminution accrue de la tension de poussée à l'écoulement. En effet, alors qu'avec le HES 200 seul, la diminution de la tension de poussée à l'écoulement est en moyenne de 75%, elle atteint 89% avec l'association HES 200 - piracétam. On a observé par ailleurs une diminution importante (17%) de l'agrégation des érythrocytes dans le cas de l'association HES 200 - piracétam, alors que cette diminution n'est seulement que de 12% avec le HES 200 seul.

En outre, on a constaté que le traitement par cette association n'exerce pas d'influence défavorable sur les concentrations des protéines plasmatiques (immunoglobuline M, alpha-2-macroglobuline, fibrinogène), ni sur la viscosité du plasma. Le traitement par l'association HES 200 - piracétam ne s'accompagne donc pas d'effets secondaires indésirables. Cette association ouvre donc de bonnes perspectives thérapeutiques dans le traitement de la claudication intermittente et en particulier dans le traitement de la claudication intermittente associée à une artériopathie oblitérante périphérique.

**Revendications pour les Etats contractants: BE, CH, DE, FR, GB, IT, LI, LU, NL, SE**

1. Compositions pharmaceutiques, caractérisées en ce qu'elles contiennent au moins un hydroxyéthyl-amidon et au moins un composé à activité nootrope.

2. Compositions pharmaceutiques selon la revendication 1, caractérisées en ce que l'hydroxyéthyl-amidon est un hydroxyéthyl-amidon de poids moléculaire compris entre 30.000 et 450.000, de préférence d'environ 200.000.

3. Compositions pharmaceutiques selon la revendication 1, caractérisées en ce que le composé à activité nootrope est un composé répondant à la formule générale

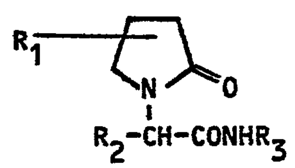

(I)

dans laquelle
$R_1$ représente un atome d'hydrogène ou un groupe hydroxyle,
$R_2$ un atome d'hydrogène ou un radical alkyle inférieur et
$R_3$ un atome d'hydrogène, le radical $-CH_2CONH_2$, le radical 2-[(bis(1méthyléthyl)amino]éthyle ou un radical de formule

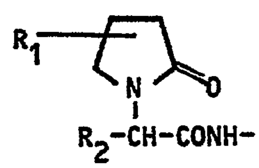

dans laquelle $R_1$ et $R_2$ ont la même signification que ci-dessus.

4. Compositions pharmaceutiques selon la revendication 1, caractérisées en ce que le composé à activité nootrope est le piracétam, l'étiracétam, le dupracétam, le pramiracétam, l'oxiracétam ou l'aniracétam.

5. Compositions pharmaceutiques selon la revendication 1, caractérisées en ce que le composé à activité nootrope est le piracétam.

6. Compositions pharmaceutiques selon l'une quelconque des revendications 1 à 5, conditionnées en vue de l'usage médical sous forme d'une solution ou suspension aqueuse.

7. Compositions pharmaceutiques selon la revendication 6, caractérisées en ce qu'elles renferment 5 à 13% en poids, de préférence 8 à 12% en poids d'hydroxyéthyl-amidon et 0,6 à 3% en poids de composé à activité nootrope.

8. Compositions pharmaceutiques selon l'une quelconque des revendications 1 à 7, caractérisées en ce que le rapport en poids entre l'hydroxyéthyl-amidon et le composé à activité nootrope est de 1,6 : 1 à 21,6 : 1, de préférence de 2,7 : 1 à 20 : 1.

9. Procédé de préparation de compositions pharmaceutiques selon la revendication 1, caractérisé en ce qu'on mélange au moins un hydroxyéthyl-amidon avec au moins un composé à activité nootrope, de préférence en solution ou suspension aqueuse.

10. Compositions pharmaceutiques selon l'une quelconque des revendications 1 à 8, pour utilisation dans le traitement de la claudication intermittente.

**Revendications pour les Etats contractants: AT , ES, GR**

1. Procédé de préparation de compositions pharmaceutiques, caractérisé en ce qu'on mélange au moins un hydroxyéthyl-amidon avec au moins un composé à activité nootrope.

2. Procédé selon la revendication 1, caractérisé en ce que l'hydroxyéthyl-amidon est un hydroxyéthyl-amidon de poids moléculaire compris entre 30.000 et 450.000, de préférence d'environ 200.000.

3. Procédé selon la revendication 1, caractérisé en ce que le composé à activité nootrope est un composé répondant à la formule générale

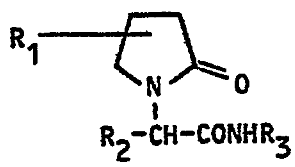

(I)

dans laquelle
$R_1$ représente un atome d'hydrogène ou un groupe hydroxyle,
$R_2$ un atome d'hydrogène ou un radical alkyle inférieur et
$R_3$ un atome d'hydrogène, le radical $-CH_2CONH_2$, le radical 2-[(bis(1-méthyléthyl)amino]éthyle ou un radical de formule

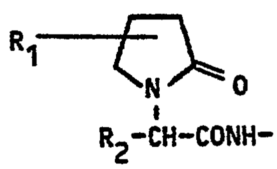

dans laquelle $R_1$ et $R_2$ ont la même signification que ci-dessus.

4. Procédé selon la revendication 1, caractérisé en ce que le composé à activité nootrope est le piracétam, l'étiracétam, le dupracétam, le pramiracétam, l'oxiracétam ou l'aniracétam.

5. Procédé selon la revendication 1, caractérisé en ce que le composé à activité nootrope est le piracétam.

6. Procédé selon l'une quelconque des revendications 1 à 5, caractérisé en ce que le mélange est effectué en solution ou suspension aqueuse.

7. Procédé selon la revendication 6, caractérisé en ce qu'on prépare une solution ou suspension aqueuse renfermant 5 à 13% en poids, de préférence 8 à 12% en poids d'hydroxyéthyl-amidon et 0,6 à 3% en poids de composé à activité nootrope.

8. Procédé selon l'une quelconque des revendications 1 à 7, caractérisé en ce que le mélange entre l'hydroxyéthyl-amidon et le composé à activité nootrope est effectué dans un rapport en poids de 1,6 : 1 à 21,6 : 1, de préférence de 2,7 : 1 à 20 : 1.

**Patentansprüche für die Vertragsstaaten: BE, CH, DE, FR, GB, IT, LI, LU, NL, SE**

1. Pharmazeutische Zusammensetzungen, dadurch gekennzeichnet, dass sie mindestens eine Hydroxyäthylstärke und mindestens eine nootropisch-wirksame Verbindung enthalten.

2. Pharmazeutische Zusammensetzungen nach Anspruch 1, dadurch gekennzeichnet, dass die Hydroxyäthylstärke eine Hydroxyäthylstärke mit einem Molekulargewicht zwischen 30.000 und 450.000, vorzugsweise ungefähr 200.000 ist.

3. Pharmazeutische Zusammensetzungen nach Anspruch 1, dadurch gekennzeichnet, dass die nootropisch-wirksame Verbindung eine Verbindung der allgemeinen Formel

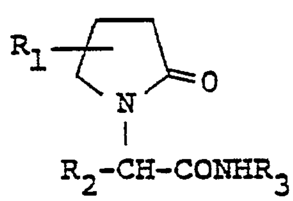

(I)

worin
$R_1$ ein Wasserstoffatom oder eine Hydroxylgruppe,
$R_2$ ein Wasserstoffatom oder einen niedrigen Alkylrest und

$R_3$ ein Wasserstoffatom, den $-CH_2CONH_2$-Rest, den 2-[(Bis(1-methyläthyl)amino]äthylrest oder einen Rest der Formel

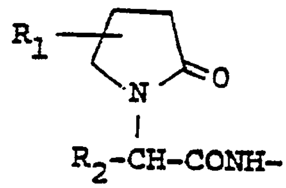

worin $R_1$ und $R_2$ die oben angegebene Bedeutung haben, darstellt.

4. Pharmazeutische Zusammensetzungen nach Anspruch 1, dadurch gekennzeichnet, dass die nootropisch-wirksame Verbindung das Piracetam, das Etiracetam, das Dupracetam, das Pramiracetam, das Oxiracetam oder das Aniracetam ist.

5. Pharmazeutische Zusammensetzungen nach Anspruch 1, dadurch gekennzeichnet, dass die nootropisch-wirksame Verbindung das Piracetam ist.

6. Pharmazeutische Zusammensetzungen nach irgendeinem der Ansprüche 1 bis 5, zubereitet im Hinblick auf medizinische Verwendung in Form einer wässrigen Lösung oder Suspension.

7. Pharmazeutische Zusammensetzungen nach Anspruch 6, dadurch gekennzeichnet, dass sie von 5 bis 13 Gew.-%, vorzugsweise von 8 bis 12 Gew.-% der Hydroxyäthylstärke und von 0,6 bis 3 Gew.-% der nootropisch-wirksamen Verbindung enthalten.

8. Pharmazeutische Zusammensetzungen nach irgendeinem der Ansprüche 1 bis 7, dadurch gekennzeichnet, dass das Gewichtsverhältnis zwischen Hydroxyäthylstärke und nootropisch-wirksamer Verbindung von 1,6 : 1 bis 21,6 : 1, vorzugsweise von 2,7 : 1 bis 20 : 1 beträgt.

9. Verfahren zur Herstellung von pharmazeutischen Zusammensetzungen nach Anspruch 1, dadurch gekennzeichnet, dass man mindestens eine Hydroxyäthylstärke mit mindestens einer nootropisch-wirksamen Verbindung, vorzugsweise in wässriger Lösung oder Suspension, mischt.

10. Pharmazeutische Zusammensetzungen nach irgendeinem der Ansprüche 1 bis 8, zur Verwendung in der Behandlung des Charcotschen Syndroms.

**Patentansprüche für die Vertragsstaaten: AT, ES, GR**

1. Verfahren zur Herstellung von pharmazeutischen Zusammensetzungen, dadurch gekennzeichnet, dass man mindestens eine Hydroxyäthylstärke mit mindestens einer nootropisch-wirksamen Verbindung mischt.

2. Verfahren nach Anspruch 1, dadurch gekennzeichnet, dass die Hydroxyäthylstärke eine Hydroxyäthylstärke mit einem Molekulargewicht zwischen 30.000 und 450.000, vorzugsweise ungefähr 200.000 ist.

3. Verfahren nach Anspruch 1, dadurch gekennzeichnet, dass die nootropisch-wirksame Verbindung eine Verbindung der allgemeinen Formel

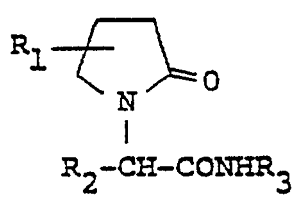

(I)

worin
$R_1$ ein Wasserstoffatom oder eine Hydroxylgruppe,
$R_2$ ein Wasserstoffatom oder einen niedrigen Alkylrest und
$R_3$ ein Wasserstoffatom, den $-CH_2CONH_2$-Rest, den 2-[(Bis(1-methyläthyl)amino]äthylrest oder einen Rest der Formel

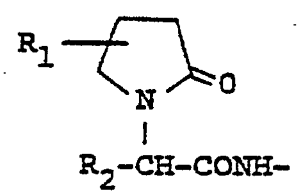

worin $R_1$ und $R_2$ die oben angegebene Bedeutung haben, darstellt.

4. Verfahren nach Anspruch 1, dadurch gekennzeichnet, dass die nootropisch-wirksame Verbindung das Piracetam, das Etiracetam, das Dupracetam, das Pramiracetam, das Oxiracetam oder das Aniracetam ist.

5. Verfahren nach Anspruch 1, dadurch gekennzeichnet, dass die nootropisch-wirksame Verbindung das Piracetam ist.

6. Verfahren nach irgendeinem der Ansprüche 1 bis 5, dadurch gekennzeichnet, dass das Mischen in wässriger Lösung oder Suspension ausgeführt wird.

7. Verfahren nach Anspruch 6, dadurch gekennzeichnet, dass man eine wässrige Lösung oder Suspension, die von 5 bis 13 Gew.-%, vorzugsweise von 8 bis 12 Gew.-% der Hydroxyäthylstärke und von 0,6 bis 3 Gew.-% der nootropisch-wirksamen Verbindung enthält, herstellt.

8. Verfahren nach irgendeinem der Ansprüche 1 bis 7, dadurch gekennzeichnet, dass das Mischen zwischen der Hydroxyäthylstärke und der nootropisch-wirksamen Verbindung in einem Gewichtsverhältnis von 1,6 : 1 bis 21,6 : 1, vorzugsweise von 2,7 : 1 bis 20 : 1, ausgeführt wird.

**Claims for the Contracting States: BE, CH, DE, FR, GB, IT, LI, LU, NL, SE**

1. Pharmaceutical compositions, characterized in that they contain at least one hydroxyethyl starch and at least one compound having nootropic activity.

2. Pharmaceutical compositions according to claim 1, characterized in that the hydroxyethyl starch having a molecular weight of from 30,000 to 450,000, preferably of about 200,000.

3. Pharmaceutical compositions according to claim 1, characterized in that the compound having nootropic activity is a compound having the general formula

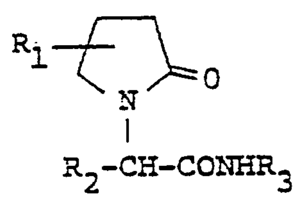

(I)

wherein

$R_1$ represents a hydrogen atom or a hydroxyl group,

$R_2$ is a hydrogen atom or a lower alkyl radical and

$R_3$ is a hydrogen atom, a $-CH_2CONH_2$ radical, a 2-[bis-(1-methylethyl)amino]ethyl radical or a radical of the formula

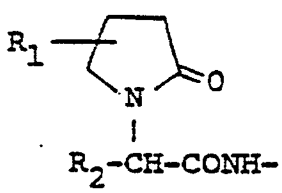

in which $R_1$ and $R_2$ have the same meanings as above.

4. Pharmaceutical compositions according to claim 1, characterized in that the compound having nootropic activity is piracetam, etiracetam, dupracetam, pramiracetam, oxiracetam or aniracetam.

5. Pharmaceutical compositions according to claim 1, characterized in that the compound having nootropic activity is piracetam.

6. Pharmaceutical compositions according to any of the claims 1 to 5, adapted for the purpose of medical use in the form of an aqueous solution or suspension.

7. Pharmaceutical compositions according to claim 6, characterized in that they contain 5 to 13% by weight, preferably 8 to 12% by weight, of hydroxyethyl starch and 0.6 to 3% by weight of a compound having nootropic activity.

8. Pharmaceutical compositions according to any of the claims 1 to 7, characterized in that the ratio by weight between the hydroxyethyl starch and the compound having nootropic activity is from 1.6 : 1 to 21.6 : 1, preferably from 2.7 : 1 to 20 : 1.

9. Process for the preparation of pharmaceutical compositions according to claim 1, characterized in that at least one hydroxyethyl starch is mixed with at least one compound having nootropic activity, preferably in aqueous solution or suspension.

10. Pharmaceutical compositions according to any of the claims 1 to 8 for use in the treatment of intermittent claudication.

**Claims for the Contracting States: AT, ES, GR**

1. Process for the preparation of pharmaceutical compositions, characterized in that at least one hydroxyethyl starch is mixed with at least one compound having nootropic activity.

2. Process according to claim 1, characterized in that the hydroxyethyl starch is a hydroxyethyl starch having a molecular weight of from 30,000 to 450,000, preferably of about 200,000.

3. Process according to claim 1, characterized in that the compound having nootropic activity is a compound having the general formula

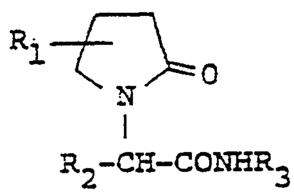

(I)

wherein
$R_1$ represents a hydrogen atom or a hydroxyl group,
$R_2$ is a hydrogen atom or a lower alkyl radical and
$R_3$ is a hydrogen atom, a $-CH_2CONH_2$ radical, a 2-[bis(1-methylethyl)amino]ethyl radical or a radical of the formula

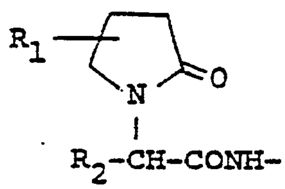

in which $R_1$ and $R_2$ have the same meanings as above.

4. Process according to claim 1, characterized in that the compound having nootropic activity is piracetam, etiracetam, dupracetam, pramiracetam, oxiracetam or aniracetam.

5. Process according to claim 1, characterized in that the compound having nootropic activity is piracetam.

6. Process according to any of the claims 1 to 5, characterized in that the mixing is effected in aqueous solution or suspension.

7. Process according to claim 6, characterized in that an aqueous solution or suspension containing 5 to 13% by weight, preferably 8 to 12% by weight of hydroxyethyl starch and 0.6 to 3% by weight of a compound having nootropic activity, is prepared.

8. Process according to any of the claims 1 to 7, characterized in that the mixing between the hydroxyethyl starch and the compound having nootropic activity is effected in a ratio by weight of from 1.6 : 1 to 21.6 : 1, preferably of from 2.7 : 1 to 20 : 1.